**Europäisches Patentamt**

**European Patent Office**

**Office européen des brevets**

(19)

(11) Numéro de publication: **0 094 278**
**B1**

(12) ## FASCICULE DE BREVET EUROPEEN

(45) Date de publication du fascicule du brevet:
**18.12.85**

(21) Numéro de dépôt: **83400809.6**

(22) Date de dépôt: **22.04.83**

(51) Int. Cl.⁴: **G 21 K 1/04,** A 61 B 6/06

(54) Diaphragme de délimitation d'un faisceau de rayonnement.

(30) Priorité: **30.04.82 FR 8207522**

(43) Date de publication de la demande:
**16.11.83 Bulletin 83/46**

(45) Mention de la délivrance du brevet:
**18.12.85 Bulletin 85/51**

(84) Etats contractants désignés:
**DE IT NL SE**

(56) Documents cités:
**FR - A - 522 956**
**FR - A - 2 113 337**
**US - A - 2 614 224**
**US - A - 3 668 402**

(73) Titulaire: **THOMSON-CSF, 173, Boulevard Haussmann, F-75379 Paris Cedex 08 (FR)**

(72) Inventeur: **Caugant, Jean, THOMSON-CSF SCPI 173, bld Haussmann, F-75379 Paris Cedex 08 (FR)**
Inventeur: **Dale, Jacques, THOMSON-CSF SCPI 173, bld Haussmann, F-75379 Paris Cedex 08 (FR)**

(74) Mandataire: **Grynwald, Albert et al, THOMSON-CSF SCPI 173, Bld Haussmann, F-75379 Paris Cedex 08 (FR)**

ACTORUM AG

## Description

L'invention concerne un diaphragme de délimitation d'un faisceau de rayonnement, notamment un faisceau de rayons X, ce diaphragme étant plus particulièrement conçu pour être placé devant l'amplificateur de luminance d'une installation de radiologie.

Une installation de radiologie comporte une source de rayons X émettant un faisceau divergent dans la direction d'un récepteur appelé amplificateur de luminance. Le patient est placé entre la source et cet amplificateur de luminance. Il est connu d'ajuster la largeur du faisceau au moyen d'un diaphragme placé entre la source et le patient. On peut ainsi limiter l'irradiation du patient à ce qui est strictement nécessaire pour l'établissement d'un diagnostic. Par ailleurs, l'amplificateur de luminance a une surface de réception à contour circulaire, dont le diamètre est déterminé en fonction de l'ouverture maximale du faisceau. Ce diamètre peut atteindre 40 cm. Lorsque l'ouverture du faisceau est limitée par le diaphragme précité à une ouverture inférieure, l'image radiologique correspondante ne se forme que sur une partie de la surface de l'amplificateur de luminance, cette partie étant toujours centrée. Dans ces conditions, l'image apparaît au milieu d'un halo de rayonnement diffusé qui nuit à sa netteté et rend son interprétation plus difficile. Il y a donc intérêt à éliminer la partie de rayonnement diffusé qui entoure l'image en plaçant une structure absorbante annulaire au-dessus de la partie non utilisée de la surface de réception de l'amplificateur de luminance.

L'invention propose un diaphragme de grande dimension susceptible de procurer le résultat recherché.

Plus précisément, l'invention concerne donc un diaphragme de délimitation d'un faisceau, notamment pour la délimitation de rayons X à l'entrée d'un récepteur d'image tel qu'un amplificateur de luminance, du type comportant un bâti dans lequel est ménagée une ouverture et une pluralité d'éléments de recouvrement partiel de ladite ouverture, caractérisé en ce que lesdits éléments de recouvrement comportent des plaques de matériau souple et que les parties arrière de celles-ci s'étendant au-delà du bord externe dudit bâti sont rabattues, sensiblement perpendiculairement au plan de ladite ouverture.

Le diaphragme défini ci-dessus est d'une structure simple et peu coûteuse et les éléments d'obturation rabattables en raison de leur souplesse permettent de réaliser un dispositif à peine plus encombrant que le diamètre de l'amplificateur de luminance au-dessus duquel il est disposé. L'adjonction d'un tel diaphragme à une installation de radiologie ne se traduit donc par aucune gêne pour le personnel hospitalier évoluant autour du patient.

Selon une caractéristique avantageuse de l'invention, les plaques de matériau souple précité sont en caoutchouc plombé.

L'invention sera mieux comprise et d'autres avantages de celle-ci apparaîtront à la lumière de la description qui va suivre d'un mode de réalisation d'un diaphragme conforme à son principe donné en référence aux dessins annexés dans lesquels:

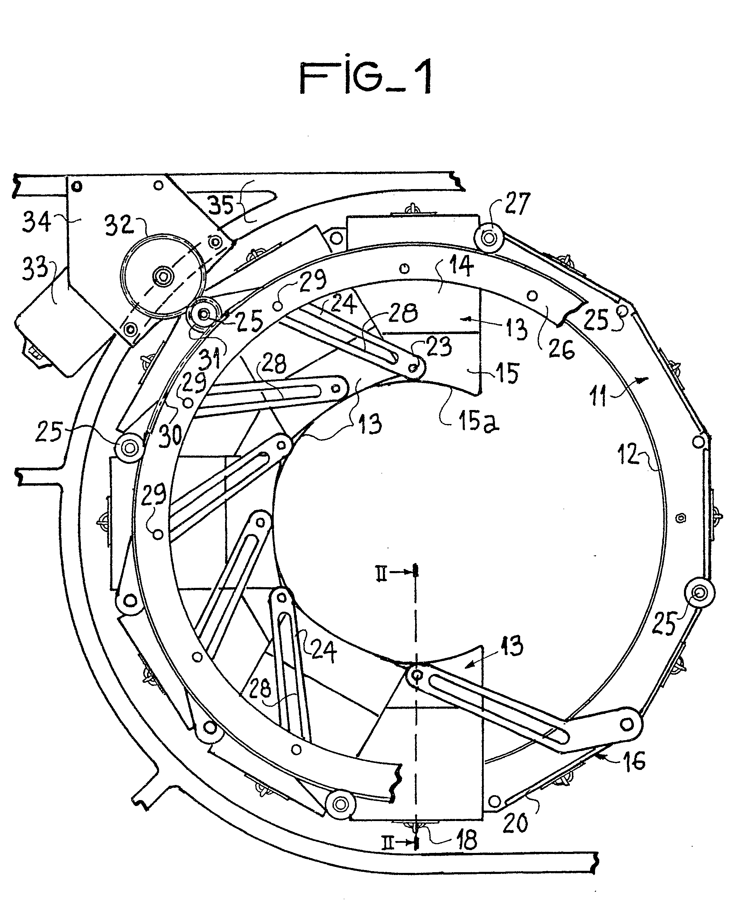

la fig. 1 est une vue de dessus avec arrachement partiel d'un diaphragme selon l'invention, certains éléments de recouvrement précités étant enlevés pour plus de clarté, et

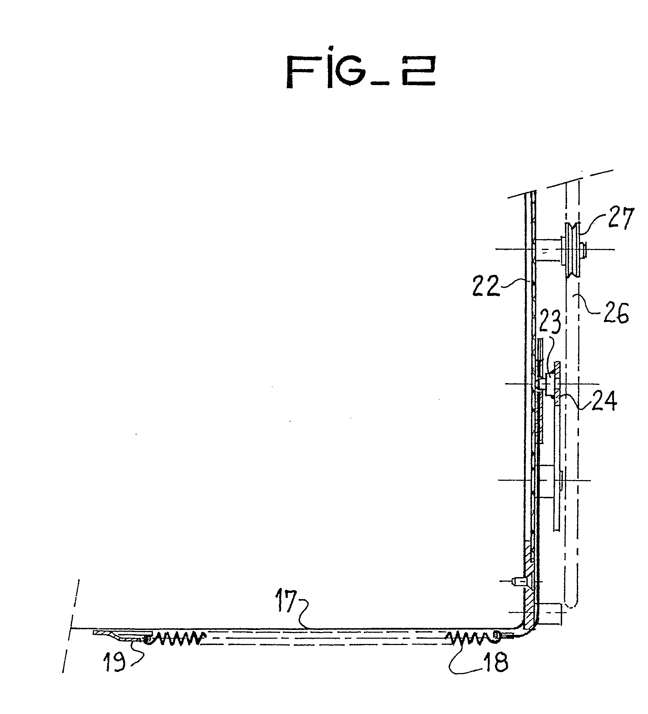

la fig. 2 est une coupe partielle II-II de la fig. 1.

En se référant aux dessins, le diaphragme représenté comporte un bâti 11 dans lequel est ménagée une large ouverture circulaire 12 et une pluralité d'éléments de recouvrement partiel 13 de l'ouverture 12. Chaque élément 13 comporte une plaque de matériau souple 14, rectangulaire, en caoutchouc plombé et une portion 15 en matériau à haute densité, en plomb dans l'exemple décrit. La portion 15 est la plus proche du centre de l'ouverture 12, elle est donc prolongée vers l'extérieur par ladite plaque de matériau souple 14. Son bord avant 15a est légèrement concave pour que l'ouverture du diaphragme soit aussi proche que possible d'un cercle. La souplesse du matériau constituant chaque plaque 14 fait que sa partie arrière qui s'étend au-delà du bord externe 16 du bâti 11 peut être facilement rabattue perpendiculairement au plan de l'ouverture 12. L'encombrement du diaphragme est donc ainsi à peine supérieur à celui de l'amplificateur de luminance qu'il est amené à recouvrir. Le bâti 11 est directement vissé à la partie supérieure de l'amplificateur de luminance 17 et les plaques de matériau souple 14 sont maintenues rabattues le long de la carrosserie de ce dernier au moyen de ressorts de tension 18 respectifs. Chaque ressort 18 est ainsi monté entre la partie arrière de chaque plaque 14 et un point fixe 19 agencé le long de la carrosserie de l'amplificateur de luminance 17. Le bord externe 16 du bâti 11 comporte autant de portions rectilignes qu'il y a de plaques de matériau souple 14. Chaque portion rectiligne est munie d'un chanfrein 20 dont la longueur est sensiblement égale à la largeur de la plaque de matériau souple 14 qui lui correspond. Ce chanfrein assure le guidage de ladite plaque lorsque celle-ci se déplace le long de la paroi latérale de l'amplificateur de luminance, lors d'un changement d'ouverture du diaphragme. Dans l'exemple illustré, le diaphragme compte 12 éléments de recouvrement et le bâti 11 a donc extérieurement la forme d'un polygone régulier à 12 côtés. Une plaque 22 en matériau transparent aux rayons X recouvre l'ouverture 12. Les éléments de recouvrement 13 glissent donc sur cette plaque et ne risquent pas de rayer la surface supérieure de l'amplificateur de luminance.

On va maintenant décrire le mécanisme qui permet d'entraîner simultanément les éléments de recouvrement 13 approximativement suivant une direction passant par le centre de l'ouverture 12. Chaque élément de recouvrement 13 est assemblé de façon articulée (axe d'articulation 23) à un bras pivotant 24 assujetti à se déplacer dans un plan parallèle à celui de l'ouverture 12, le bras étant articulé autour d'un tourillon 25 respectif, fixé au bâti 11 et orienté perpendiculairement à la surface de l'ouverture 12. On compte donc 12 tourillons 25 répartis régulièrement tout autour du bâti 11 au voisinage des sommets du polygone mentionné ci-dessus. Les bras pivotants 24 sont couplés à une couronne 26 montée mobile en rotation, coaxialement à l'ouverture circulaire 12. Bien entendu, l'axe de rotation de cette couronne est fictif. Son guidage en rotation est assuré au moyen de plusieurs poulies 27 montées en rotation libre autour de certains des tourillons 25. La bordure circulaire externe de la couronne 26 est engagée dans les gorges de ces poulies. Ces dernières assurent donc à la fois le maintien de la couronne 26 en position au-dessus des bras pivotants 24 et le guidage de celle-ci en rotation. Chaque bras 24 est muni d'une lumière 28 et un pion 29 solidaire de la couronne 26 est engagé dans chaque lumière. La bordure externe de la couronne 26 comporte également un secteur denté 30 en prise avec un pignon 31 lui-même monté sur l'un des tourillons 25. Le pignon 31 engrène un pignon 32 entraîné en rotation au moyen d'un moteur électrique 33. Ce dernier est solidaire d'une platine 34 fixée à une carcasse moulée 35 existant dans une installation de radiologie et entourant l'amplificateur de luminance pour assurer sa protection.

Le fonctionnement du diaphragme selon l'invention découle avec évidence de la description qui précède. La mise en rotation de la couronne 26 fait pivoter tous les bras 24 de la même façon du fait de la coopération des pions d'entraînement 29 et des lumières 28. Les éléments de recouvrement 13 sont donc tous déplacés de la même façon suivant un arc de cercle très proche d'un rayon de l'ouverture 12, compte tenu de la position du point de pivotement d'un bras 24 par rapport à l'élément de recouvrement auquel il est accouplé. Les ressorts 18 maintiennent les parties non utilisées des plaques de matériau souple parallèlement à la carcasse de l'amplificateur de luminance; ils assurent aussi une certaine tension sur lesdites plaques. Il est possible de prévoir un copiage de position entre le diaphragme situé au niveau de la source de rayons X et celui qui vient d'être décrit. Pour ce faire, un potentiomètre pourrait être mécaniquement couplé au pignon 31.

Bien entendu, l'invention n'est pas limitée au mode de réalisation spécifique qui vient d'être décrit mais comprend tous les équivalents techniques des moyens mis en jeu.

**Revendications**

1. Diaphragme de délimitation d'un faisceau, notamment pour la délimitation d'un faisceau de rayons X à l'entrée d'un récepteur d'image tel qu'un amplificateur de luminance, du type comportant un bâti (11) dans lequel est ménagée une ouverture (12) et une pluralité d'éléments de recouvrement partiel (13) de ladite ouverture, caractérisé en ce que lesdits éléments de recouvrement comportent des plaques de matériau souple (14) et que les parties arrière de celles-ci s'étendant au-delà du bord externe (16) dudit bâti sont rabattues, sensiblement perpendiculairement au plan de ladite ouverture (12).

2. Diaphragme selon la revendication 1, caractérisé en ce que lesdites plaques de matériau souple (14) sont en caoutchouc plombé.

3. Diaphragme selon l'une des revendications 1 ou 2, caractérisé en ce que la portion (15) de chaque élément de recouvrement la plus proche du centre de ladite ouverture (12) est en matériau à haute densité, par exemple en plomb, cette portion étant prolongée vers l'extérieur par une plaque de matériau souple (14) précitée.

4. Diaphragme selon l'une des revendications 1 à 3, caractérisé en ce que le bord externe (16) précité dudit bâti comporte autant de portions rectilignes qu'il y a de plaques de matériau souple et que chaque portion rectiligne est chanfreinée (20) pour assurer le guidage d'une plaque.

5. Diaphragme selon l'une des revendications précédentes, caractérisé en ce qu'un ressort de tension (18) est monté entre ladite partie arrière de chaque plaque (13) et un point fixe (19) correspondant.

6. Diaphragme selon l'une des revendications précédentes, caractérisé en ce qu'il comporte un mécanisme (24, 25, 26, 30, 31, 32, 33) pour entraîner simultanément les éléments de recouvrement en suivant au moins approximativement une direction passant approximativement par le centre de symétrie de ladite ouverture (12).

7. Diaphragme selon la revendication 6, du type à ouverture (12) circulaire, caractérisé en ce que chaque élément de recouvrement est assemblé de façon articulée (23) à un bras pivotant (24) assujetti à se déplacer dans un plan parallèle à celui de ladite ouverture (12).

8. Diaphragme selon la revendication 7, caractérisé en ce que lesdits bras pivotants (24) sont couplés à une couronne (26) montée mobile en rotation coaxialement à ladite ouverture circulaire, entre des poulies de guidage (27) ou des moyens analogues, montées en rotation libre sur ledit bâti (11).

9. Diaphragme selon la revendication 8, caractérisé en ce que chaque bras pivotant (24) est muni d'une lumière (28) et qu'un pion d'entraînement (29) solidaire de ladite couronne est engagé dans cette lumière.

10. Diaphragme selon l'une des revendications 8 ou 9, caractérisé en ce que le bord externe de ladite couronne (26) comporte un secteur denté (30) en prise avec au moins un pignon (31) entraîné par un moteur (33).

**Patentansprüche**

1. Begrenzungsblende für einen Strahl, insbesondere zur Begrenzung eines Röntgenstrahls am Eingang eines Bildempfängers, z. B. eines Leuchtkraftverstärkers, mit einem Gestell (11), in dem eine Öffnung (12) vorgesehen ist, und mit zahlreichen Elementen (13) zur partiellen Abdeckung dieser Öffnung, dadurch gekennzeichnet, dass diese Abdeckungselemente Platten (14) aus nachgiebigem Material enthalten und dass die rückwärtigen Bereiche dieser Platten, die über den äusseren Rand (16) des Gestells vorstehen, im wesentlichen senkrecht zur Ebene der Öffnung (12) umgebogen sind.

2. Blende nach Anspruch 1, dadurch gekennzeichnet, dass die Platten aus nachgiebigem Material (14) aus mit Blei befrachtetem Kautschuk bestehen.

3. Blende nach einem der beiden Ansprüche 1 oder 2, dadurch gekennzeichnet, dass der Bereich (15) jedes Abdeckungselements, der dem Zentrum der Öffnung (14) am nächsten liegt, aus einem Material grosser Dichte, beispielsweise Blei, besteht und sich nach aussen in einer Platte aus nachgiebigem Material (14) fortsetzt.

4. Blende nach einem der Ansprüche 1 bis 3, dadurch gekennzeichnet, dass der genannte äussere Rand (16) des Gestells ebensoviele geradlinige Bereiche aufweist, wie es Platten aus nachgiebigem Material gibt, und dass jeder geradlinige Bereich eine Schrägkante (20) zur Führung einer Platte besitzt.

5. Blende nach einem der vorhergehenden Ansprüche, dadurch gekennzeichnet, dass eine Zugfeder (18) zwischen dem rückwärtigen Bereich jeder Platte (13) und einem entsprechenden festen Punkt (19) montiert ist.

6. Blende nach einem der vorhergehenden Ansprüche, dadurch gekennzeichnet, dass sie einen Mechanismus (24, 25, 26, 30, 31, 32, 33) zum simultanen Antrieb der Abdeckungselemente enthält, wobei mindestens angenähert eine Richtung verfolgt wird, die ungefähr durch das Symmetriezentrum der Öffnung (12) verläuft.

7. Blende nach Anspruch 6, vom Typ kreisförmige Öffnung (12), dadurch gekennzeichnet, dass jedes Abdeckungselement an einem Schwenkarm (24) angelenkt (23) ist, der so befestigt ist, dass er sich in einer zur Ebene der Öffnung (12) parallelen Ebene bewegt.

8. Blende nach Anspruch 7, dadurch gekennzeichnet, dass die Schwenkarme (24) an einen Ring (26) angekoppelt sind, der koaxial zur kreisförmigen Öffnung und in Drehrichtung beweglich zwischen Führungsrollen (27) oder analogen Mitteln montiert ist, wobei diese Führungsrollen frei drehend auf dem Gestell (11) montiert sind.

9. Blende nach Anspruch 8, dadurch gekennzeichnet, dass jeder Schwenkarm (24) ein Langloch (28) aufweist und dass ein Antriebsstift (29), der auf dem Ring sitzt, in dieses Langloch eingreift.

10. Blende nach einem der beiden Ansprüche 8 oder 9, dadurch gekennzeichnet, dass der Aussenrand des Rings (26) einen mit Zähnen versehenen Sektor (30) aufweist, der mit mindestens einem von einem Motor (33) angetriebenen Ritzel (31) in Eingriff steht.

## Claims

1. A diaphragm for delimiting a beam, particularly for delimiting an X-ray beam at the input of an image receiver such as an image intensifier, of the type comprising a frame (11) in which an opening (12) is provided, and a plurality of elements (13), which partly overlap said opening, characterized in that said overlap elements comprise plates made of resilient material (14), and that the rear portions of these plates extending beyond the outer edge (16) of said frame are turned down substantially at a right angle with respect to the plane of said opening (12).

2. A diaphragm according to Claim 1, characterized in that said plates of resilient material (14) are made from lead rubber.

3. A diaphragm according to Claim 1 or 2, characterized in that the portion (15) of each overlap element which is closest to the center of the opening (12) is made of a high density material, for example lead, this portion being followed outwardly by a said plate of resilient material (14).

4. A diaphragm according to one of Claims 1 to 3, characterized in that said outer edge (16) of the frame comprises as many straight portions as there are plates of resilient material, and that each straight portion is chamfered (20) in order to ensure the guidance of a plate.

5. A diaphragm according to one of the preceding claims, characterized in that an extension spring (18) is mounted between said rear part of each plate (13) and a corresponding fixed point (19).

6. A diaphragm according to one of the preceding claims, characterized in that it includes a mechanism (24, 25, 26, 30, 31, 32, 33) for simultaneously driving the overlap elements whilst following at least approximately a direction passing approximately through the center of symmetry of said opening (12).

7. A diaphragm according to Claim 6 of the type having a circular opening (12), characterized in that each overlap element is assembled in an articulated manner (23) with a pivoting arm (24) which is forced to move in a plane parallel to that of said opening (12).

8. A diaphragm according to Claim 7, characterized in that the pivoting arms (24) are coupled to a ring (26) which is mounted to rotate coaxially to said circular opening between guidance pulleys (27) or analog means, which rotate freely on said frame (11).

9. A diaphragm according to Claim 8, characterized in that each pivoting arm (24) is provided with an opening (28) and that a drive pin (29) integral with said ring is engaged in said opening.

10. A diaphragm according to Claims 8 or 9, characterized in that the outer edge of said ring (26) comprises a toothed segment (30) engaging with at least one pignon (31) driven by a motor (33).

# FIG_1

# FIG_2